# EUROPEAN PATENT APPLICATION

(11) **EP 1 016 389 A1**
(43) Date of publication of application: **05.07.2000**
(21) Application number: 98124825.5
(22) Date of filing: 30.12.1998
(51) Int. Cl.: A61F 13/15

(54) **Tridimensional disposable absorbent article with an absorbent core having special fluid handling properties**

(71) Applicant: THE PROCTER & GAMBLE COMPANY, Cincinnati, Ohio 45202 (US)
(72) Inventor: Wierlacher, Stefan, 65122 Pescara (IT); Sierri, Giancarlo, 65105 Montesilvano (IT)
(74) Representative: Hirsch, Uwe Thomas M.H.

(57) **Abstract**

A disposable absorbent article adapted to form a tridimensional shape prior to use with a generally upwardly convex rear region, having a body facing surface and a garment facing surface, a longitudinal symmetry plane, a front end edge and a rear end edge, and comprising a liquid pervious topsheet, a backsheet joined to said topsheet and an absorbent core intermediate the backsheet and the topsheet. The absorbent core has an improved fluid handling capability in order to increase fluid acquisition and distribution, while preventing side leakage at the same time.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to disposable absorbent sanitary napkins, catamenials, incontinence inserts, and pantiliners that are adapted to form a three dimensional shape before use, in order to provide an enhanced fit for the body and reduced leakage by means of said tridimensional shape that matches the non-planar surfaces and the non-linear grooves of the body. The disposable absorbent articles of the present invention comprise an absorbent core which has an improved fluid handling capability in order to increase fluid acquisition and distribution, while preventing side leakage at the same time.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

Major disadvantages of known disposable absorbent articles intended to be worn externally of the body, e.g. leakage, wet/dirty feeling, discomfort, are related to the poor body fit achieved by these articles. With respect to e.g. sanitary napkins, different attempts have been made in order to provide such articles with the capability of conforming to the body anatomy. Body conforming absorbent articles are well known in the art, both those that are initially flat, and that are adapted to form a three dimensional shape before use in order to match the wearer's anatomy, for example by incorporating lines of preferential folding, and by being formed into the preferred three dimensional shape by the user by means of manipulation prior to their application to the body, and those that are provided already shaped prior to use in the desired three dimensional shape.

In European patent application No. 97122739.2 filed on 23 December 1997, entitled "Tridimensional disposable absorbent article having a slit in the rear region" (P&G case CM1667Q), a tridimensional disposable absorbent article is described which has a tridimensional shape prior to use and comprises an upwardly convex rear region preferably having an inverted V shaped longitudinal ridge. The absorbent article also comprises a slit or cut in the rear region which extends from the rear end edge and is oriented substantially longitudinally. The rear cut or slit gives the preferably inverted V shaped rear portion of the absorbent article the capability of bending around an axis which is perpendicular to the symmetry plane of the article, in order to better fit the various body shapes, specifically in the area of the gluteal groove, where the rear region of the absorbent article preferably extends in order to provide a better protection. Preferably the tridimensional disposable absorbent article can be applied directly to the users body, rather than being applied first to the panty prior to wearing the panty itself with the absorbent article attached thereon.

The tridimensional disposable absorbent article described in EP application No. 97122739.2 has the capability of better adjusting its shape, particularly its upwardly convex rear region, to the different anatomies taking into account the possible interactions with the undergarment, and any variations experienced during the wearing time, due e.g. to the wearer's movements, at the same time providing a better fit and a proper positioning of the rear region interested by the rear slit or cut with respect to the preferred acquisition region of the absorbent article.

Disposable absorbent articles adapted to form a three dimensional shape before use, particularly those being already shaped prior to use such as those described in the above mentioned application, do provide a good fit to the anatomy, and therefore a better comfort to the user and a reduced leakage, but can still be improved in their fluid handling capabilities.

Disposable absorbent articles of the above described type, particularly those that are intended to be applied directly to the body before a panty is worn, can in fact achieve a very good fit with the users anatomy, and, by staying fit and close to the anatomy, are therefore capable of receiving fluid in a well defined area of the absorbent core through the fluid pervious body contacting element.

Two apparently contrasting needs in this type of articles are an improvement in the fluid handling capability, particularly in terms of fluid acquisition and distribution capacity of the absorbent core, in combination with a better control against side leakage. Starting from a circumscribed and typically well defined acquisition area the absorbed fluid, usually released in multiple gushes, shall be in fact effectively directed over a larger area of the absorbent core, at the same time providing a barrier at the longitudinal edges of the core in order to avoid side leakage.

The provision of e.g. a fluid distribution strip in the core which is narrower than the core itself, as it is known in the art, might reduce the occurrence of side leakage, but certainly also reduces the area over which the fluid can be distributed starting from the acquisition zone, particularly in case of a circumscribed, and possibly narrow acquisition zone.

Also known methods of providing the article with liquid impervious side barriers to prevent liquid from being lost or squeezed from the longitudinal edges of the core, e.g. by closing the longitudinal edges with fluid impervious elements, or alternatively by providing a topsheet which is selectively made liquid impervious outside the acquisition area, and typically along the longitudinal edges of the core, do not solve completely the problem of side leakage.

Such barriers in fact on one hand reduce the area of the body facing surface of the article that can actually acquire fluid, and on the other hand do not provide any protection against fluid running off the surface of the topsheet, e.g. owing to particularly heavy gushes that may not be quickly acquired into the absorbent structure. This last effect is even more evident in articles adapted to form a three dimensional shape before use which fits the user's anatomy, or already three dimensionally shaped prior to use, such as those described in the above mentioned EP application No. 97122739.2, which typically have a convex shape, e.g. an inverted V shape, in the central and rear region of the article, comprising the area where fluid is most likely to be acquired into the absorbent core. In case of heavy gushes not immediately acquired into the absorbent structure at least part of the fluid may in fact run off the sloping body facing surface of this type of articles and towards the longitudinal edges. The provision of an absorbent core which reduces the risk of side leakage also due to the possible run off of fluid, especially in case of sudden gushes, over the convex shaped surface of the acquisition area of the article can therefore be highly beneficial in the context of the preferred disposable absorbent articles adapted to form a three dimensional shape before use.

It is therefore an object of the present invention to provide a disposable absorbent article adapted to form a three dimensional shape before use, which has an absorbent core with a better fluid handling capability in order to increase the fluid acquisition and distribution capacity, at the same time reducing the occurrence of side leakage, specifically in presence of sudden fluid gushes.

It is another object of the present invention to provide a disposable absorbent article as defined above having a core with a storage layer and fluid barriers along the longitudinal edges thereof which can separate the topsheet from the fluid storage layer, being at the same time capable of acquiring fluid gushes, and of releasing them to the fluid storage layer.

These and other advantages of the invention will become apparent from the following description.

### SUMMARY OF THE INVENTION

The present invention reefers to a disposable absorbent article adapted to form a three dimensional shape before use, comprising a liquid pervious topsheet, a liquid impervious backsheet, and an absorbent core intermediate the topsheet and the backsheet. The absorbent core has a central acquisition region and a peripheral region external to the central acquisition region, and comprises a fluid storage layer. The absorbent core is capable of acquiring body fluids through the topsheet in the central acquisition region of the absorbent core. The absorbent core further comprises in the peripheral region a material distinct from the fluid storage layer, wherein this material is a high void volume fluid handling material.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of one embodiment of a sanitary napkin according to the present invention, seen from the side thereof that faces the wearer in use;
FIG. 2 is a cross-sectional view of the sanitary napkin of FIG. 1 on line 2-2;
FIG. 3 is a top view of the sanitary napkin of FIG. 1;
FIG. 4 is a curve taken from an anatomical section of the body of a wearer, which schematically represents the central non linear groove of the female anatomy as seen in lateral direction;
FIGS. 5a, 5b, and 5c are cross-sectional views of the sanitary napkin of FIG. 1 on lines 5a-5a, 5b-5b, and 5c-5c, respectively, comprising a schematical representation of the absorbent core, which does not show the details of the structure according to the present invention;
FIG. 6 is a perspective view of the sanitary napkin of FIG. 1, seen from the side that lies remote from the wearer in use;
FIGS. 7a and 7b are perspective views of a sanitary napkin according to the present invention similar to that illustrated in FIGS. 1 to 6, showing two in use positions of a different embodiment of the present invention;
FIG. 8 is a cross sectional view of a sanitary napkin of the present invention similar to that illustrated in FIG. 5c, but showing the details of an absorbent core structure according to an embodiment of the present invention;
FIG. 9 is a cross sectional view similar to that illustrated in FIG. 8, showing an alternative preferred core structure.

### DETAILED DESCRIPTION OF THE INVENTION

The present invention relates to disposable absorbent articles that are adapted to form a three dimensional shape before use. This definition refers to articles which are already provided with a three dimensional shape prior to use, and also comprises articles which are initially flat, but incorporate features that allow the article to assume a preferred three dimensional shape before use, e.g. being shaped by the user by means of manipulation prior to wear them. Such features can comprise e.g. preferential folding lines impressed in the article structure with known means, for example by embossment, bonding, or sealing. Such articles according to the present invention are distinct from purely flat conventional disposable absorbent articles that, when compressed and squeezed during the use, in any case assume a shape that can be said three dimensional as compared to the initial flat state, but are not adapted to form a preferred three dimensional shape before use.

Preferably, the present invention relates to disposable absorbent articles already provided with a tridimensional shape prior to use, and more preferably having an upwardly convex, most preferably transversely inverted V shaped rear region, which exhibit absorbency for bodily fluids, the protection of the users garments from soiling, and improved physical comfort to the user, which are also easy to produce and to package. According to a particularly preferred embodiment of the present invention that will be described hereinbelow, such articles are provided in said upwardly convex rear region with at least a cut or a slit extending from the rear end edge substantially in longitudinal direction. Said at least one cut or slit defines cut edges in the rear region of the article that are allowed to move apart in order to provide enhanced fit to the body and better conformability to the wearers anatomy, particularly in the rear region, where the article typically extends through the posterior perineal area towards the groove between the buttocks. Such tridimensional disposable absorbent articles are preferably also provided with a tridimensional structure capable of matching the non-linear grooves and the non-planar surfaces of the female body. Unless otherwise stated, by simply saying "tridimensional absorbent article" is meant herein a disposable absorbent article adapted to form a three dimensional shape before the use, as explained above. The tridimensional disposable absorbent articles are described below by reference to a sanitary napkin or catamenial.

The term "sanitary napkin", as used herein, refers to an article which is worn by females externally of the body and adjacent to the pudendal region and which is intended to absorb and contain the various body fluids which are discharged from the body (e.g., vaginal discharges, menses, and/or urine) and which is intended to be discarded after a single use. It should be understood, however, that the present invention is also applicable to other feminine hygiene or catamenial pads such as pantiliners, or other absorbent articles such as incontinence pads, and the like.

The term "use", as used herein, refers to the period of time that starts when the absorbent article is actually put in contact with the anatomy, and of course comprises the period during which the article is worn.

The terms "joined" or "affixed", as used herein, encompasses configurations whereby a first member is directly connected to a second member and configurations whereby a first member is indirectly connected to a second member by connecting the first member to intermediate members which in turn are connected to the second member.

As used herein, the term "pudendal" refers to the externally visible female genitalia and is limited to the labia majora, the labia minora, the clitoris, and the vestibule.

According to the present invention, the disposable absorbent article comprises an absorbent core having a central acquisition region, where the fluid is to be acquired after exiting the body, and a peripheral region external to the central acquisition region. The absorbent core has a fluid storage layer and a high void volume fluid handling material distinct from the fluid storage layer in at least part of the peripheral region.

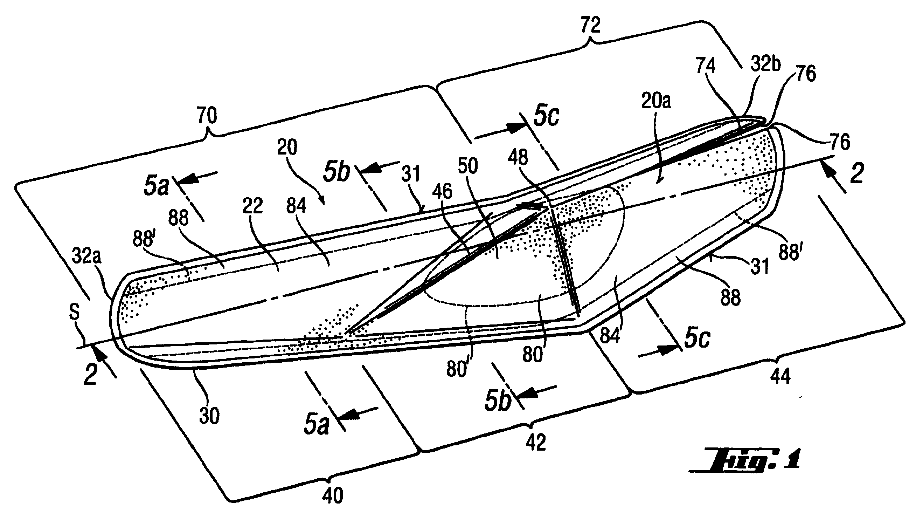

FIG. 1 is a perspective view of a preferred sanitary napkin 20 of the present invention with its preferred tridimensional structure before use, with most of the portion of the sanitary napkin 20 that faces or contacts the wearer, oriented towards the viewer. By saying "before use", it is meant that the preferred sanitary napkin 20 of the present invention is provided with a tridimensional structure before it is actually worn. The sanitary napkin can nevertheless be packaged in a folded flat configuration, being subsequently unfolded to get the tridimensional shape just before wearing it. As better shown in FIG. 2, the sanitary napkin 20 comprises a liquid pervious topsheet 22, a liquid impervious backsheet 23 joined with the topsheet 22, and an absorbent core 24 positioned between the topsheet 22 and the backsheet 23.

The sanitary napkin 20 has two surfaces, a body facing or contacting surface 20a and a garment facing or contacting surface 20b. The body contacting surface 20a is intended to be worn adjacent to the body of the wearer while the garment surface 20b is on the opposite side and is intended to be directed towards the undergarment when the sanitary napkin 20 is worn, e.g. placed against it. Corresponding body facing and garment facing surfaces can also be identified in each single layer that constitutes the sanitary napkin 20, e.g., in the absorbent core 24. The sanitary napkin 20 has a longitudinal symmetry plane S. The term "longitudinal", as used herein, refers to a line, axis or direction in the sanitary napkin 20 that is generally aligned with (e.g., approximately parallel to) a vertical plane which bisects a standing wearer into left and right body halves when the sanitary napkin 20 is worn. The symmetry plane S of the sanitary napkin 20 substantially corresponds to this vertical plane that bisects the standing wearer. While it is preferred that the sanitary napkin 20 is exactly divided by the longitudinal symmetry plane S into two symmetrically equal halves, it is not excluded that the two halves be not specular. The term "transverse", as used herein, refers to a direction that is generally perpendicular to the longitudinal symmetry plane. The term "longitudinally oriented" refers to a direction, as seen in plan view, comprised within ±45 degrees, of the longitudinal symmetry plane S; the term "transversely oriented" similarly refers to any other direction, as seen in plan view.

The terms "front" and "rear", as used herein, refer to portions or edges in the sanitary napkin 20 that are oriented towards the front and rear part of the wearer's body, respectively, when the sanitary napkin 20 is being worn.

The sanitary napkin 20 has a periphery 30, that is defined by the outer edges of the sanitary napkin 20. The longitudinal edges 31 of the sanitary napkin 20 are aligned with the longitudinal symmetry plane S, and the ends edges of the sanitary napkin 20 comprise a front end edge 32a and a rear end edge 32b. The absorbent core 24 of the sanitary napkin has a front portion 40, a central portion 42 and a rear portion 44, each one preferably corresponding to approximately one third of the total length of the absorbent core 24. A front region 70 and a rear region 72 are identified in the sanitary napkin 20, respectively comprising at least the front portion 40 and at least the rear portion 44 of the absorbent core 24.

In the preferred embodiment of the present invention the tridimensional sanitary napkin 20 is already provided prior to use with a tridimensional structure that is intended to match the complex body shapes of the female anatomy. The tridimensional structure has more preferably a structural tridimensionality, by "structural tridimensionality" being meant that the structure cannot be completely flattened, i.e. spread out, onto a flat surface while keeping its integrity, that is, without being in any case e.g. torn, crushed or squeezed. In other words, the tridimensional structure cannot be achieved by simply folding or pleating an initially flat article, but is inherently owned by the absorbent article according to the present invention. The tridimensional sanitary napkin 20 of the present invention has preferably a substantially constant thickness, that is more preferably less than 5 mm; the sanitary napkin can be therefore considered of the thin type.

While the topsheet, the backsheet, and the absorbent core may be assembled in a variety of well known configurations (including so called "tube" products or side flap products), FIG. 1 shows a preferred embodiment of the sanitary napkin 20 in which the topsheet 22 and the backsheet 23 have length and width dimensions generally larger than those of the absorbent core 24. The topsheet 22 and the backsheet 23 extend beyond the edges of the absorbent core 24 to thereby form the periphery 30 of the sanitary napkin 20.

The topsheet 22 is compliant, soft feeling, and non-irritating to the wearer's skin. Further, the topsheet 22 is liquid pervious, permitting liquid (e.g. menses and/or urine) to readily penetrate through its thickness. A suitable topsheet 22 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in U.S. Patent Application Serial No. 07/794,745, Aziz et al., filed on November 19, 1991. Alternatively, the body surface of the topsheet can be made hydrophilic by treating it with a surfactant such as is described in U.S. 4,950,254.

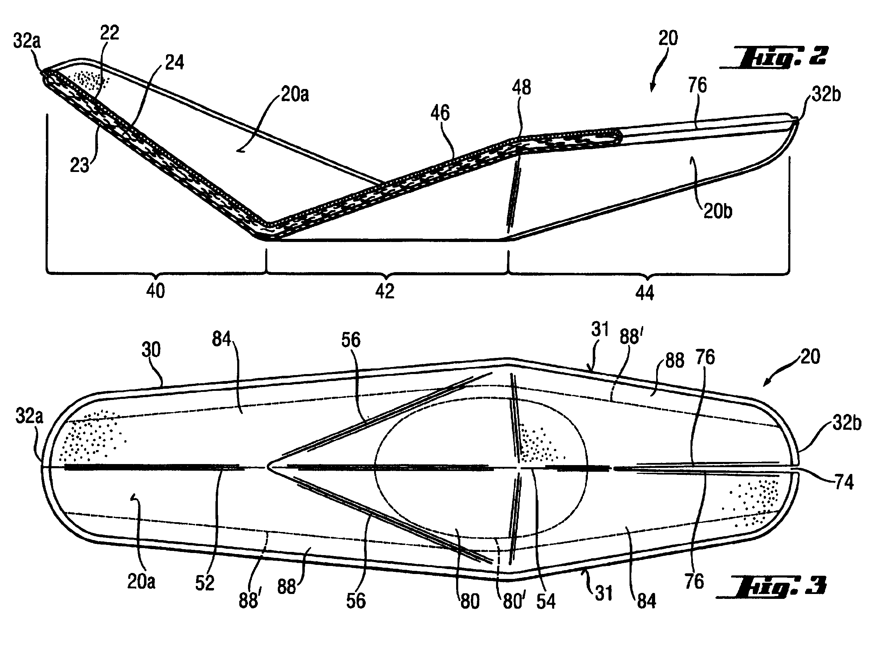

The absorbent core 24 comprises any absorbent means that is capable of absorbing or retaining liquids (e.g., menses and/or urine). The absorbent core 24 may be manufactured in a wide variety of sizes and shapes (e.g., rectangular, oval, hourglass, asymmetric, etc.). The absorbent core 24 comprises a fluid storage layer 90 which is intended for fluid absorption and retention. According to the present invention the absorbent core 24 has a central acquisition region 80 in which it most likely acquires fluid from the body through the topsheet 22. As shown in FIGS. 1 and 3, where the central acquisition region 80 is shown actually on the body facing surface of the absorbent article 20 and is delimited by a dotted line 80' for clarity, the central acquisition region 80 can have for example an oval shape, being centered along the longitudinal symmetry plane S and aligned with it, and comprising part of the central portion 42 and of the rear portion 44 of the absorbent core 24. The central acquisition region 80 does not extend transversely up to the longitudinal edges 82 of the core, as can be better seen in FIG. 8. The absorbent core 24 also has a peripheral region 84 which is external to the central acquisition region 80, and actually corresponds to the remaining portion of the absorbent core 24, separate from the central acquisition region 80.

According to the present invention the absorbent core 24 comprises a high void volume fluid handling material 86 in at least part of the peripheral region 84. The high void volume fluid handling material 86 is distinct from the fluid storage layer 90.

By the term "distinct", as used herein, it is broadly meant something that is different from something else, in terms of e.g. structure, component materials, or physical characteristics. Distinct can also correspond to "physically separate". This definition includes for example an absorbent core 24 made of a same material having different characteristics, e.g. different bulkiness, in the central acquisition region 80 as compared to the peripheral region 84, therefore providing a fluid storage layer and a high void volume fluid handling material distinct from the fluid storage layer according to the present invention.

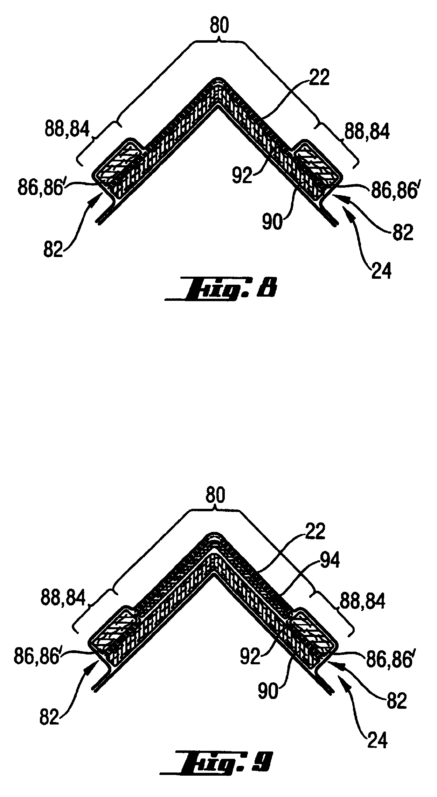

In the preferred embodiment illustrated in FIGS. 1 and 3, and as better seen in FIGS. 8 and 9, the absorbent core 24 has in the peripheral region 84 a side region 88 located along each of the longitudinal edges 82, delimited for clarity in FIGS. 1 and 3 by dotted lines 88' on the body facing surface of the article 20, which may run substantially parallel to the longitudinal edges 82 of the core 24. The high void volume fluid handling material 86 is comprised only in the side regions 88, as a pair of high void volume fluid handling structures 86', each one substantially having the same extension of the respective side region 88. In the preferred embodiment of the present invention the side regions 88, and the corresponding high void volume fluid handling structures 86', have a substantially constant width, measured from the respective longitudinal edges 82 of the core 24, which for an absorbent article such as the sanitary napkin 20 illustrated in FIGS. 1 to 9, can typically range from 5 mm to 15 mm, with a preferred value of about 10 mm.

In the preferred embodiment illustrated in FIG. 8 the fluid storage layer 90 is actually a component of the absorbent core 24 which is separate from the high void volume fluid handling structures 86'. The fluid storage layer 90 typically has the whole extension of the absorbent core 24, with the two high void volume fluid handling structures 86' comprised between the topsheet 22 and the storage layer 90, and in fluid communication therewith. The high void volume fluid handling structures 86' have a thickness such as to separate the topsheet 22 from the fluid storage layer 90. Typically the thickness of the high void volume fluid handling structure 86' ranges from 1 mm to 5 mm, preferably between 1.5 mm and 2.5 mm.

The high void volume fluid handling material 86 can be constituted by any preferably hydrophilic material having a void volume which is sufficiently high to freely receive fluid, and at the same time with a low capillarity, and therefore low wicking force, such that any liquid acquired into the structure is not diffused or retained, but rather easily released to a typically denser absorbent structure in fluid communication with it. The void volume can be expressed in terms of bulkiness measured under a pressure of 2 kPa. According to the present invention, the bulkiness of the high void volume fluid handling structure 86 is preferably at least 15 cm³/g, preferably comprised between 20 cm³/g and 100 cm³/g, more preferably comprised between 30 cm³/g and 50 cm³/g, as measured under a pressure of 2 kPa. It is believed that the high bulkiness of the fluid handling material 86 is such that the fluid is free to flow with very little impedance within this material.

In a preferred embodiment of the present invention illustrated in FIG. 8 the absorbent core 24 preferably further comprises a fluid acquisition layer 92 which is at least partially coextensive with both the fluid storage layer 90 and the high void volume fluid handling material 86, therefore extending in the central acquisition region 80 and in at least part of the peripheral region 84. The fluid acquisition layer 90 is intermediate the topsheet 22 and the fluid storage layer 90, and in fluid communication therewith, in the central acquisition region 80, and is also intermediate the high void volume fluid handling material 86, namely each of the two the high void volume fluid handling structures 86', and the fluid storage layer 90 where it is coextensive with them, i.e. in at least part of the peripheral region 84. In the preferred embodiment of the present invention the fluid acquisition layer 92 has about the same extension of the fluid storage layer 90, i.e., about the whole extension of the absorbent core 24.

Suitable materials for the high void volume fluid handling material 86 are those having the required features in terms of bulkiness. Fibrous materials, preferably comprising synthetic fibres, or foam structures can be used for the high void volume fluid handling material 86. In a preferred embodiment of the present invention the high void volume fluid handling material 86 comprises a dry laid, for example an airlaid, web of staple fibres having a bulkiness of at least 15 cm³/g, as measured under a pressure of 2 kPa. The high void volume fluid handling material 86 is preferably hydrophilic. Examples of preferred fibrous materials having high bulkiness and useful as the high void volume fluid handling materials of the present invention are described in PCT application WO 94/28838 as the dry laid webs of staple fibres used as a secondary sheet for absorbent articles.

Preferably, the high void volume fluid handling material 86 should also possess a good resistance to collapse and a good resilience in wet state, evaluated according to the Wet Collapse and Wet Resilience test methods described in the above mentioned PCT application WO 94/28838. This implies that the high void volume fluid handling material 86 can preferably withstand the pressure exerted on it during the use with a low deformation, and preferably can also effectively recover the initial shape/thickness once the pressure is released.

The fluid acquisition layer 92 can comprise any structure having a good fluid acquisition combined with fluid transmission capacity, this latter preferably in both longitudinal and transverse direction, such as e.g. low basis weight nonwoven materials. Suitable materials can comprise woven or nonwoven layers, such as for example spunlaced nonwovens, preferably hydrophilic.

The fluid storage layer 90 can be made from a wide variety of liquid-absorbent materials commonly used in sanitary napkins and other absorbent articles such as comminuted wood pulp that is generally referred to as airfelt. Examples of other suitable absorbent materials include creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993), absorbent sponges, synthetic staple fibres, polymeric fibres, hydrogel-forming polymer gelling agents, peat moss, tissue including tissue wraps and tissue laminates, or any equivalent materials or combinations of materials. Suitable fluid storage layers comprising foams are described in European Applications 0 598 833, 0 598 823 and 0 598 834. Suitable absorbent structures, useful as fluid storage layers to be incorporated in an absorbent core according to the present invention, and comprising tissue laminates with particles of hydrogel-forming polymer gelling agents comprised therebetween are described in International Patent Applications WO 94/01069 and WO 95/17868.

The configuration and construction of the absorbent core may also be varied without losing the advantages related to the specific features of the absorbent core according to the present invention and described so far (e.g., the absorbent core may have varying caliper zones, e.g., profiled so as to be thicker in the centre, hydrophilic gradients, superabsorbent gradients). Of course the total absorbent capacity of the absorbent core should, however, be compatible with the design loading and the intended use of the sanitary napkin. Further, the overall size and absorbent capacity of the absorbent core may be varied to accommodate different uses such as incontinence pads, pantiliners, regular sanitary napkins, or overnight sanitary napkins. Preferably the absorbent articles of the present invention are sanitary napkins which are uniform in thickness.

The backsheet 23 and the topsheet 22 are positioned adjacent the garment facing surface 20b and the body facing surface 20a, respectively, of the absorbent core 24 and are preferably joined thereto and to each other by attachment means (not shown) such as those well known in the art. For example, the backsheet 23 and/or the topsheet 22 may be secured to the absorbent core 24 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zieker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 23 is impervious to liquids (e.g., menses and/or urine) and is preferably manufactured from a thin plastic film, although other flexible liquid impervious materials can also be used. In use, the backsheet 23 is interposed between the absorbent core 24 and the users undergarments. The function of the backsheet 23 is to prevent exudates which may be expelled from or which inadvertently bypass the absorbent core 24 from contacting and soiling the user's undergarments. The backsheet 23 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet 23 is preferably embossed and/or matte finished to provide a more clothlike appearance. Further, the backsheet 23 may permit vapours to escape from the absorbent core 24 (i.e., it can be breathable) while still preventing exudates from passing through the backsheet 23.

As illustrated in FIGS. 1 and 2, the preferred tridimensional sanitary napkin 20 in its preferred embodiment has before use a tridimensional structure with a longitudinal oriented ridge 50 in the central and rear portions 42, 44 of the absorbent core 24, such that the line of intersection 46 of the longitudinal symmetry plane S with the body facing surface 20a has a slope decreasing rearwardly, i.e. towards the rear end edge 32b, in the central portion 42 and in the rear portion 44 of the absorbent core 24. This can be seen more clearly in FIG. 2, where the longitudinal sectional view of the sanitary napkin 20 shows the line of intersection 46 with its decreasing slope in the central and rear portions 42, 44.

According to a most preferred embodiment of the present invention the sanitary napkin 20 further comprises a cut or slit 74 in its rear region 72, extending from the rear end edge 32b and, in the preferred embodiment illustrated in FIGS. 1 to 3, being aligned with the longitudinal symmetry plane S. The cut or slit 74 can affect the whole structure of the sanitary napkin 20, i.e., in the illustrated embodiment, the topsheet 22, the absorbent core 24 and the backsheet 23, so defining corresponding cut edges 76. It is preferred that the structure of the sanitary napkin 20 is sealed along the cut edges 76, e.g. by joining together the topsheet 22 and the backsheet 23 by means of adhesive, or heat, or any other known means, so that portions of the cut absorbent core 24 are not exposed along the cut edges 76. The cut or slit 74 allows the cut edges 76 of the sanitary napkin 20 to move apart form each other along said cut or slit 74, so that the longitudinally oriented, preferably inverted V shaped structure of the ridge 50 can bend at any point along the cut or slit 74 around an axis that is perpendicular to the symmetry plane S, as better shown in FIGS. 7a and 7b, where a slightly different embodiment of the present invention is illustrated, in order to better fit the body anatomy typically in the region of the gluteal groove, where the rear region 72 of the tridimensional sanitary napkin 20 preferably extends in order to provide a better rearward protection.

More in detail, FIGS. 7a and 7b illustrate the same sanitary napkin 20 in two different embodiments of the rear region 72, corresponding to two different wearing situations, where the longitudinally oriented, inverted V shaped structure of the ridge 50 bends at two different points around an axis perpendicular to the symmetry plane S. This can be due to the adaptation of the article to different anatomies, i.e. to wearers having different body shape in the region of the gluteal groove, e.g., FIG. 7b could show the configuration of a sanitary napkin 20 while worn by a smaller wearer. Or, alternatively, this can be caused by e.g. different forces acting on the sanitary napkin during the use.

The cut or slit 74 therefore provides the structure of the inverted V shaped ridge 50, which would be per se less capable of bending around an axis perpendicular to the symmetry plane S without creasing and/or bending away form the body, and/or exerting a force on the remaining portions of the sanitary napkin 20 extending forward of said rear region 72, with the capability of adapting to the various body shapes, particularly in the region of the gluteal groove, therefore adjusting to different groove lengths and radii of curvature, and also to changes that typically occur with time in the same wearer, e.g. through movements.

In the particularly preferred embodiment of the present invention illustrated in FIGS. 1 to 7, where a sanitary napkin 20 having a three dimensional shape already formed prior to use is shown, the line of intersection 46 has a preferred profile with a rearwardly decreasing slope as seen in cross-sectional view, as will be explained more in detail below.

The decreasing slope of said line of intersection 46 can be expressed mathematically if said line of intersection 46 is considered in a Cartesian x-y system lying in the symmetry plane S, wherein the x-axis is defined by the two points of intersection of the longitudinal symmetry plane S with the front end edge 32a and the rear end edge 32b of the sanitary napkin 20, substantially corresponding to the points indicated by numerals 32a and 32b in the cross-section view of the sanitary napkin 20 illustrated in FIG. 2, and wherein the body facing surface 20a faces towards positive y values.

With respect to this system of axes one can form the first derivative of the line of intersection 46. According to the present invention, the first derivative of this line 46 in the longitudinal direction has at least one value that is larger in the central portion 42 of the absorbent core 24 than at least one value in the rear portion 44 of the absorbent core 24. This includes the preferred case, illustrated in FIGS. 1 and 2, where the intersection line 46 is always inclined upward towards the rear end edge 32b with two different slopes in the central portion 42 and in the rear portion 44, and also alternative embodiments in which, e.g., the line of intersection 46 is inclined upward in the central portion 42 and downward in the rear portion 44.

The consecutive values of the first derivative of the line of intersection 46 can decrease continuously towards the rear end edge 32b, implying that the line of intersection 46 has a curved profile with a continuously decreasing slope, or, alternatively, the first derivative can assume different discrete values along the length of the intersection line 46. For example, it can be constant in either the central portion 42, or in the rear portion 44, or in both, the latter being the case of the embodiment illustrated in FIGS. 1 and 2, where the intersection line 46 is formed by two substantially rectilinear portions having constant slopes, with a slope change at a point 48 of the line of intersection 46 positioned where the central portion 42 of the absorbent core 24 merges the rear portion 44.

In the preferred embodiment of the present invention illustrated in FIGS. 1 to 3 the cut or slit 74 extends from the rear end edge 32b substantially along the symmetry plane S. Therefore no actual line of intersection 46 can be identified where the cut edges 76 are moved apart form each other, but only where no cut or slit exists, or also where the cut edges 76 are kept close to each other and no displacement of them occurs. This is typically the case of the preferred tridimensional sanitary napkin 20 of the present invention since the bending axis perpendicular to the symmetry plane S can move along the cut or slit 74 according to the different anatomies and/or to the changes experienced during the wearing time.

In the preferred embodiment of the present invention herein described the sanitary napkin 20 features a line of intersection 46 with the preferred profile, that is kept with any possible bent configuration of the rear portion 72 since the cut or slit 74 does not extend up to the point 48 where the slope change in the line of intersection 46 occurs. In other words, even if the cut edges 76 of the rear region 72 of the sanitary napkin 20 are caused to move apart along the entire length of the cut or slit 74, the longitudinal oriented ridge 50 still comprises a line of intersection 46 with the preferred slope decreasing rearwardly. In this preferred embodiment the sanitary napkin 20 therefore keeps its most preferred structural tridimensionality wherever the bending axis perpendicular to the symmetry plane S is located along the cut or slit 74 in the rear region 72.

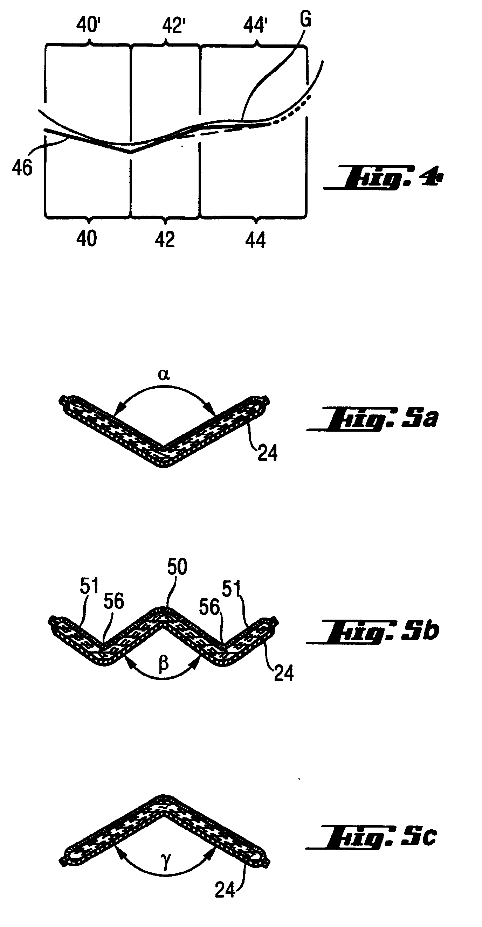

A line of intersection 46 with the above described profile in combination with the cut or slit 74 in the rear region 72 provides the preferred sanitary napkin 20 of the present invention with a longitudinally oriented ridge 50 in the central and rear portions 42, 44 of the absorbent core 24. The ridge 50 has a longitudinal non linear profile that is intended to match in use the central non linear groove of the female anatomy extending from the labia majora to the perineum and into the gluteal groove, and having approximately the shape schematically indicated in the corresponding central and rear portions 42', 44' of the curve G, also featuring a corresponding front portion 40', illustrated in FIG. 4, where the matching profile of a line of intersection 46 in a sanitary napkin illustrated in FIGS. 1 to 3 is also shown. The cut or slit 74 in the rear region 72 also provides the longitudinally oriented ridge 50 with the capability of bending around an axis perpendicular to the symmetry plane S and positioned at any location along the cut or slit 74 itself, in order to better fit the different body anatomies and the varying in use conditions in the region of the gluteal groove.

The dotted line following the rearward portion of the profile of the line of intersection 46 in FIG. 4 corresponds to the portion of the rear cut or slit 74 where the cut edges 76 are actually displaced from each other during the use of the sanitary napkin 20 of the present invention. Such dotted line does not correspond to an intersection line since, as already explained, there can be no intersection between the symmetry plane S and the body facing surface 20a where the cut edges are caused to move apart. It rather indicates the actual profile described by one of the cut edges 76 in the rear region 72 of the sanitary napkin 20 as seen in cross-sectional view, where the upwardly convex, inverted V shaped rear region of the sanitary napkin 20 is allowed to bend along an axis perpendicular to the symmetry plane S in order to better fit the body anatomy in the region of the gluteal groove, corresponding to the rearmost part of the rear portion 44' of the curve G. The profile shown in FIG. 4 can be considered as substantially corresponding to a sanitary napkin having the configuration illustrated in FIG. 7a, with the bending axis perpendicular to the symmetry plane S located closer to the rear end edge 32b.

The profile of the longitudinally oriented ridge 50 as defined by the line of intersection 46 with its slope decreasing rearwardly, and in combination with the cut or slit 74 in the rear region 72 can provide the sanitary napkin 20 with an improved fit to the wearer's body. In the preferred embodiment illustrated in FIG. 1, when going from front to rear, the forward portion of the ridge 50, with a substantially constant slope, is intended to fit the groove between the labia majora. The subsequent portion of the ridge 50 that bridges the central and the rear portions 42, 44 of the absorbent core 24, with its change in slope, has a profile that is capable of matching in use the downwardly concave portion of the central non linear groove of the female anatomy in the region going from the rearward part of the labia majora to the perineum, so as to achieve a continuous contact with the body. This provides for a better comfort and for a more effective interception of the fluids as they are released from the body. The rearward portion of the longitudinally oriented ridge 50, still belonging to the rear portion 44 of the absorbent core 24 and having a constant slope in the embodiment of FIG. 1, is intended to extend between the buttocks in the gluteal groove. Owing to its slighter slope, as compared to the forward portion of the ridge, it is capable of contacting the body without causing any stress between the anatomy and this portion of the sanitary napkin, which could in turn cause discomfort, and/or prevent the desired substantially continuous contact between the ridge 50 and the wearer's anatomy along the entire length of the non linear groove extending from the labia majora up to the gluteal groove. Finally, the rearmost portion of the ridge 50, characterized by the presence of the cut or slit 74, can bend, typically upwardly, around an axis perpendicular to the symmetry plane S, in order to better fit the body shape in the area of the gluteal groove, where the rear region 72 of the sanitary napkin 20 preferably extends to provide an increased protection, e.g. against rear leakage that can be experienced during motion or during the sleep, when the wearer lies on her back. This provides for an improved fit of the sanitary napkin 20 in the area of the gluteal groove, also avoiding any possible negative interaction with the undergarment, that could otherwise exert a force on the upwardly convex rear region of the sanitary napkin 20 extending rearwardly, therefore causing said upwardly convex region to crease or move away from the body.

A ridge 50 with a preferred profile having a slope decreasing rearwardly can get further into the non linear groove of the female anatomy, as schematically indicated in FIG. 4. The ridge 50 with the profile indicated by the line 46 is in fact capable of following the profile of the groove, indicated by the curve G, by extending past a line, indicated with the dashed line in FIG. 4, that connects two points along the central groove of the body surface where the sanitary napkin has contact with the anatomy, e.g. the two points where the sanitary napkin contacts the body in correspondence of the forward and rearward portions of the ridge. The rear end of the dashed line actually goes up to the rearmost portion of the ridge 50 where the cut edges 76 are displaced form each other; it therefore corresponds to the beginning of the dotted line, as explained hereinbefore, following the profile of the line of intersection 46. A ridge shaped with a linear profile as those known in the art cannot extend past this line, since such a ridge substantially corresponds to this line, and hence cannot provide a continuous contact with the body along the entire length of the ridge.

Of course the situation described so far of a preferred sanitary napkin 20 having a preferred three dimensional shape already formed prior to use, and its interaction with the wearer's anatomy represents only a particular preferred embodiment of the present invention. This embodiment is intended to indicate the general capability of the ridge 50 with the preferred longitudinal non linear profile, in combination with the rear cut or slit 74, to match in use along its entire length the central non linear groove of the female anatomy, therefore providing for a better contact with the body and an increased comfort. The specific structure of the absorbent core 24 according to the present invention, preferably together with the preferred three dimensional shape described herein, provides for a better management of the fluid acquired within the article, by means of an increased fluid acquisition and distribution capacity of the core 24, and a better protection against side leakage.

In the preferred embodiment of the present invention illustrated in FIGS. 1 and 2 the tridimensional sanitary napkin 20 preferably has a low constant thickness that is less than 5 mm, wherein the preferred tridimensional structure is provided without the use of humps or of regions of different thickness, and it is an inherent feature of the sanitary napkin 20, rather than an added feature, achieved e.g. by bending, folding or joining together an initially planar structure. This constitutes a preferred embodiment of the present invention, that actually requires that the absorbent article is adapted to form the three dimensional shape before use. The present invention therefore comprises alternative embodiments in which the desired three dimensional shape is achieved in an e.g. initially flat sanitary napkin before use, for example by means of lines of preferential folding included in the structure, and activated by manipulation by the user herself prior to actually wearing the article.

As shown in the preferred embodiment of the present invention illustrated in FIGS. 1 and 2, the front portion 40 of the absorbent core 24 is preferably upwardly concave, in order to better conform to the wearer's anatomy in the region of the mons pubis.

The preferred three dimensional sanitary napkin 20 illustrated in FIGS. 1 and 2 shows a particularly preferred configuration for the front, central and rear portions 40, 42, and 44 of the absorbent core 24. As viewed in transverse section the front, central and rear portions of the absorbent core 24 have respectively a V shape, a W shape, and an inverted V shape, as better shown in FIGS. 5a, 5b, and 5c, where transverse sections of the sanitary napkin 20 taken on lines 5a-5a, 5b-5b, and 5c-5c respectively of FIG. 1 are illustrated. It has to be noted that in FIGS. 5a, 5b, and 5c the absorbent core identified with the reference numeral 24 is illustrated only schematically, and does not actually show the details of the structure described so far and according to the present invention.

The different transverse shapes provide the preferred sanitary napkin 20 with the further capability of conforming to the wearer's anatomy in a direction substantially perpendicular to the already defined symmetry plane S. The V shape of the front portion 40 and the inverted V shape of the rear portion 44 merge together gradually in the central portion 42, where the resulting W shape is predisposed to fit the area of the labia majora and of the perineum. In use, the longitudinally oriented ridge 50 is intended to fit the longitudinal central groove as above described, while the side portions 51 bent upwardly can match the groin creases, i.e. the two grooves that are formed between the body and the legs, typically in the area where the panty elastics contact the body.

In the particularly preferred embodiment of the present invention illustrated in FIGS. 1 and 2 the preferred three dimensional sanitary napkin 20 is provided with an increased capability of conforming to the wearer's anatomy than that given by simpler differentiated transverse shaping of the different portions of the absorbent core 24.

The preferred tridimensional structure of the sanitary napkin 20, preferably already present in the article prior to use, is such that the width of the angle γ of the inverted V shaped portion increases towards the rear end edge 32b of the sanitary napkin 20 starting from a minimum preferred value at a position corresponding to the merging of the rear portion 44 with the central portion 42 of the absorbent core 24, where it substantially corresponds to the angle β of the central inverted V part of the W shaped central portion 42, which is in turn substantially constant along the entire length of this portion 42. Therefore the rearward portion of the ridge 50, typically positioned in use between the buttocks, can more easily widen its inverted V shape during the wearing of the product without being restrained, so providing the sanitary napkin with a better conformability to the anatomical configuration of the wearer. Of course the further preferred feature of the cut or slit 74 in the rear region 72 of the sanitary napkin 20, not shown in FIG. 5c, which is a section taken at a location where the cut or slit 72 is not present, allows the cut edges 76 of the rearmost portion of the ridge 50 to move apart form each other to even better fit the region of the gluteal groove, so adapting to different lengths and radii of curvature of said groove.

A feature similar to that described for the rear portion 44 is preferably provided in the V shaped front portion 40 of the absorbent core 24, where the angle α of the V increases its width towards the front end edge 32a of the sanitary napkin 20 from a minimum preferred value at a point corresponding to the merging of the front portion 40 with the central portion 42. This will allow the portion of the sanitary napkin 20 which is closer to the front end edge 32a to more easily flatten in transverse direction during wearing in order to accommodate the relatively flat front part of the mons pubis, while still providing an overall concave shape to effectively follow the surface of the mons pubis.

The angles of the V shaped front portion 40 and/or of the inverted V shaped rear portion 44 of the absorbent core 24, and consequently of the entire sanitary napkin 20, can therefore increase towards respective end edges 32a and/or 32b up to values around 180°, in order to better accommodate the anatomy of the wearer without inducing any substantial stress in the structure, thus providing for a better fit and comfort.

The preferred feature of the angles increasing towards respective end edges in the V shaped and inverted V shaped portions is achieved by giving the front portion 40 and/or the rear portion 44 of the absorbent core 24 a cup shaped structure with any means known to the man skilled in the art. For example, in the preferred sanitary napkin 20 of the present invention illustrated in FIGS. 1 and 2 this is achieved by cutting away a narrow V shaped portion of material centered along the longitudinal centreline of initially flat front portion 40 and rear portion 44 of the absorbent core 24, and of the topsheet 22 and the backsheet 23 as well, and having substantially the same length of the front portion 40 and of the rear portion 44, and then joining together the cut edges with known means, e.g. by thermobonding, along the junction lines identified as 52 and 54 in FIG. 3. Of course the junction line 54 does not extend up to the rear end edge 32b when the preferred cut or slit 74 has to be formed. The final preferred tridimensional structure illustrated in FIGS. 1 and 2 is then achieved by suitably bending the non planar sanitary napkin 20, e.g. along lines of preferential bending, formed in the absorbent core 24 by means of e.g. embossments or partial cuts, such as the embossments 56 in FIG. 3, as can be readily determined by the man skilled in the art. In this preferred embodiment the cup shaped structure of the central and rear portions 42, 44 of the absorbent core 24, and therefore of the corresponding rear region 72 of the sanitary napkin 20, is intrinsically stable, i.e., has the already defined structural tridimensionality that is not hindered by the rear cut or slit 74, since it does not run the whole length of the rear portion 44 of the absorbent core 24 up to the peak 48.

The presence of the above described preferred feature in the preferred sanitary napkin of the present invention illustrated in FIGS. 1 and 2 can be readily ascertained when folding transversely the sanitary napkin 20 in order to superimpose the front portion 40 or the rear portion 44 of the absorbent core over the central portion 42 along a fold line that approximately in the unfolded sanitary napkin corresponds to a line separating respectively the front portion 40 or the rear portion 44 from the central portion 42: in both cases the folding line will show an angle rather than being rectilinear.

The combination of the preferred structure of the sanitary napkin 20 of the present invention, having a preferred three dimensional shape already formed prior to use, and comprising the longitudinally oriented ridge 50 with the preferred profile of the line of intersection 46, with the rear cut or slit 74 in the rear region 72, provides the sanitary napkin 20 with an increased capability to fit to the non-planar surfaces and the non-linear grooves of the female anatomy, along the entire length of the sanitary napkin. The improved fit achieved by a sanitary napkin which is adapted to form a three dimensional shape before use, particularly a sanitary napkin having a three dimensional shape already formed prior to use, such as in the preferred embodiment described so far, is capable of providing a proper and stable positioning of the sanitary napkin during the use. There is therefore no risk that the sanitary napkin is mispositioned with respect to the anatomy, or is moved from its preferred location during the use. This provides that in use the liquid is properly received and acquired in an acquisition zone corresponding to the central acquisition region 80 of the absorbent core 24. In a preferred absorbent article as the sanitary napkin 20 described so far, having the preferred three dimensional shape prior to use, and properly positioned against the users body, the liquid will actually reach the absorbent core 24 in a rather circumscribed area comprised in the central acquisition region 80, typically located in the central portion 42 of the absorbent core 24, and more likely forward of the peak 48 of the ridge 50, which roughly corresponds to the centre of the oval central acquisition region 80 of FIGS. 1 and 3.

During the use of a sanitary napkin 20 according to the present invention, represented by the preferred embodiment described so far, fluid is acquired through the topsheet 22 into the absorbent core 24 at a point which may be considered to be substantially located along the ridge of the inverted V shaped section of the absorbent core 24. This can be seen by way of indication only in FIG. 8.

It has in fact to be noted that the transverse section of the absorbent article 20 actually illustrated in FIG. 8 is taken along line 5c of FIG. 1, and therefore does not necessarily correspond to the transverse section of the absorbent article 20 which is nearer to the point where more likely the fluid will reach the absorbent core, which can be rather located forward of the peak 48 of the ridge 50 as explained above. However, the following description will be done with reference to FIG. 8 for simplicity reasons.

As illustrated in FIG. 8, the absorbent core 24 preferably also comprises the fluid acquisition layer 92. The fluid therefore is acquired through the topsheet 22 into this layer 92 which, as explained above, has a high acquisition speed and is capable of effectively distributing the fluid on a larger surface both in longitudinal and in transverse direction. The fluid acquired and distributed in the acquisition layer 92 can be therefore easily transferred into the adjacent underlying fluid storage layer 90 which in the preferred embodiment of FIG. 8 is substantially totally coextensive with the fluid acquisition layer 92. As the fluid gets closer to each of the longitudinal edges 82 of the absorbent core by diffusing within the thickness of the fluid acquisition layer 92, and reaches the corresponding side region 88, it is separated from the topsheet 22 by the respective high void volume fluid handling structure 86' which is interposed between the fluid acquisition layer 92 and the topsheet 22. The high void volume fluid handling structure 86' in fact has a thickness such that it separates the topsheet 22 from the fluid storage layer 90. Moreover, owing to its high volume and low capillarity, the high void volume fluid handling material 86 of the structures 86' has a very low wicking force and therefore is not likely to acquire the fluid from the underlying layer which is in fluid communication with it, i.e., the fluid acquisition layer 92 in the illustrated embodiment of FIG 8, or, alternatively, in a less preferred embodiment, the fluid storage layer 90 or any portion of the absorbent core 24 distinct from the high void volume fluid handling material 86.

Side leakage due to fluid reaching the longitudinal edges 82 of the absorbent core by means of liquid capillary diffusion within a layer having the desired good acquisition and diffusion capacity is therefore avoided by this insulating effect performed by the high volume fluid handling structure 86.

The risk of side leakage due to this diffusion mechanism can be further reduced if, as it is preferred, the fluid acquisition layer 92 has an extension in transverse direction which is slightly smaller than the overall extension of the absorbent core 24, i.e., if it does not actually reach at least the longitudinal edges 82 of the absorbent core 24. The longitudinal edges 82 are therefore identified in this preferred embodiment by the superimposed corresponding longitudinal edges of the storage layer 90 and of the respective high void volume fluid handling structures 86'.

The high void volume fluid handling material 86 also performs a second effect which is particularly important in case of sudden gushes of fluid onto an absorbent article having a preferred three dimensional shape before use.

When, during the use of the sanitary napkin, the absorbent core receives a sudden gush of fluid which is particularly heavy, the fluid which is not promptly acquired through the topsheet 22 into the absorbent structure, namely into the fluid acquisition layer 92 in the preferred embodiment of FIG. 8, tends to run off the body facing surface of the fluid acquiring layer 92. This is more likely to occur in case of an absorbent product adapted to form a three dimensional shape before use, such as the preferred sanitary napkin 20 of FIG. 8, owing to the inverted V shape and to the body facing surface of the fluid handling layer 92 sloping down towards the longitudinal side edges 82 of the absorbent core. Fluid running off the body facing surface of the fluid acquisition layer 92, and more in general of the absorbent core 24, can therefore get close to any of the longitudinal edges 82 of the absorbent core 24. However, as soon as the fluid run off reaches any of the high void volume fluid handling structures 86' in the respective side region 88 it is acquired into the void spaces of the high void volume fluid handling material 86 and side leakage is therefore prevented. The thickness of the high void volume fluid handling structure 86' also contributes to this damming effect, in that it preferably creates a step on the body facing surface of the absorbent core 24 that can help intercept fluid run off.

The specific features of the high void volume fluid handling material 86 in terms of high bulkiness, and preferably also of resistance to collapse and resilience in wet state, allow the high void volume fluid handling material 86 to acquire rapidly fluid runoffs into the open space, and to release it subsequently almost completely to the underlying absorbent layer, namely the fluid acquisition layer 92 in the preferred embodiment of FIG. 8, owing to its low wicking force. The high void volume fluid handling material 86 is also capable of temporarily acquiring fluid that can be occasionally squeezed out of the fluid acquisition layer 92 and/or the fluid storage layer 90 in case the sanitary napkin is subjected to particularly heavy stresses or pressures during the use, for example in combination with heavy fluid loads, while still keeping the capacity of subsequently releasing said fluid back to the absorbent structure of the core 24.

The high void volume fluid handling material 86 therefore acts as a semi-permeable barrier that closes the longitudinal side edges 82 of the core 24 in order to avoid fluid squeeze-out from adjacent absorbent layers, and at the same time provides the absorbent article with excellent gush acquisition properties to avoid side leakage due to fluid runoff, owing to its open structure and to its preferred hydrophilicity. Moreover, the high void volume fluid handling material 86 also separates the topsheet 22 from the underlying absorbent structure of the core 24 along the side regions 88. This not only prevents side leakage, but also keeps the absorbed fluid far from the body facing surface of the article in these regions, even when the underlying absorbent layers contain absorbed fluids, or even become saturated with fluid up to the longitudinal edges 82 in case of particularly heavy loads. This is due both to the low wicking force and to the relatively high caliper of the high void volume fluid handling material 86, comprised in the core 24 as the high void volume fluid handling structures 86'.

In a preferred embodiment of the present invention such as that illustrated in FIG. 8, the absorbent core 24 comprises a fluid acquisition layer 92 constituted by a spunlaced nonwoven such as that sold by Suominen under the tradename Fibrella 2200 NBC-FB. The storage layer 90 is constituted by a melt bonded/latex bonded fibrous web comprising cellulose and bicomponent fibres and also particles of hydrogel forming polymeric material, such as that sold by Buckeye Corporation under the tradename of Vizorb x404-h70. The high void volume fluid handling material 86 of the high void volume fluid handling structures 86' consists of an airlaid web of synthetic bicomponent PE-PP staple fibres having a diameter of about 22 µm and a length of about 12 mm, said web having a basis weight of 60 g/m² and a bulkiness of 33.3 cm³/g.

In an alternative preferred embodiment of the present invention, illustrated in FIG. 9 where corresponding elements as in FIG. 8 are indicated with the same numerals, the absorbent core 24 also comprises a fluid distribution layer 94 which extends at least in the central acquisition region 80. As illustrated in FIG. 9, the fluid distribution layer 94 preferably extends over the whole central acquisition region 80 and also in part of the peripheral region 84 of the absorbent core, but preferably not in the side regions 88, and is interposed between the fluid storage layer 90 and the fluid acquisition layer 92. The fluid distribution layer 94 has a density which is typically higher than the density of the high void volume fluid handling structure 86, and preferably has the capability of distributing the fluid preferentially in longitudinal direction, for example by comprising a fibrous structure oriented in this direction. Preferred materials for the fluid distribution layer are for example airlaid or wetlaid thermally or chemically bonded fibrous materials having the desired fluid distribution properties. Absorbent foams such as those already mentioned can also be used. In a preferred embodiment of the present invention the fluid distribution layer comprises a thermal bonded wet laid structure comprising 90% of cellulose fibres, and 10% of bicomponent fibres having a diameter of 20 µm with a density of about 0.09 g/cm³, and a caliper of about 0.9 mm.

The fluid distribution layer 94 has the scope of distributing the fluid received from the fluid acquisition layer 92 preferentially in longitudinal direction owing to its narrower width. The fluid distribution layer 94 combined with the fluid acquisition layer 92 provide the absorbent article with an effective fluid distribution capability lengthwise without compromising the acquisition speed given by the fluid acquisition layer 92.

The absorbent core can also be ring rolled in order to increase its softness and flexibility according to U.S. Pat. No. 4,107,364, U.S. Pat. No. 4,834,741, U.S. Pat. No. 5,143,679, U.S. Pat. No. 5,156,793, U.S. Pat. No. 5,167,897.

According to a further alternative embodiment of the present invention, the longitudinal side edges 82 of the absorbent core 24 can also be closed by means of a liquid impervious material, e.g. by portions of the backsheet 23 folded upwardly against said side edges 82, but of course not reaching the body facing surface of the absorbent core 24, in order to further increase the already good protection against side leakage provided by the absorbent article of the present invention.

The provision of a central acquisition region 80 for the fluid which is substantially far from the rear end edge 32b of the sanitary napkin is also relevant in the preferred embodiment of the present invention described with reference to FIGS. 1 to 3, characterized by the slit or cut 74 positioned rearwardly of this central acquisition region. The region of the preferred absorbent article comprising the cut or slit 74 is in fact not assumed to receive any fluid directly from the body. Moreover, the cut or slit 74, being oriented substantially longitudinally, does not create any obstacle to the diffusion of the liquid within the absorbent core 24, which itself occurs in a preferred path oriented in longitudinal direction.

The rear cut or slit 74 can therefore be left completely open, with no need of an additional material joining the cut edges 76.

In an alternative preferred embodiment of the present invention, however, illustrated in FIGS. 7a and 7b, the sanitary napkin 20 can also comprise a material 78 that joins the cut edges 76 of the rear cut or slit 74, and that allows the cut edges 76 to move apart. Said material 78 is preferably liquid impervious, therefore providing the article with an added protection in the rear region 72 with the cut or slit 74. Said material 78 shall be provided by any known means with the capability of allowing the cut edges 76 to move apart, e.g. it can be extensible, elastic, or pleated, as illustrated in FIGS. 7a and 7b, where a liquid impervious plastic film 78 provided with pleats 80 and joining the cut edges 76 along their entire length is shown. The material 78 can be a separate element added to the structure of the sanitary napkin 20, e.g. joined to the backsheet 24, or can be integral with the structure, being e.g. a portion of the backsheet 24 made extensible by known means.

In the preferred embodiments of the present invention shown in FIGS. 1 to 7 the sanitary napkin 20 always comprises a single rear slit or cut 74 in its rear region 72, extending from the rear end edge 32b substantially along the longitudinal symmetry plane S. In alternative embodiments of the present invention more than one cut or slit can be provided in the rear region of a tridimensional absorbent article. In further alternative embodiments the at least one cut or slit does not necessarily run along the longitudinal symmetry plane S of the tridimensional absorbent article, provided that the at least one cut or slit extends from the rear end edge in a direction towards a point located on the longitudinal symmetry plane S.

An alternative embodiment of the present invention preferably comprising a tridimensional shape prior to use similar to that illustrated in FIGS. 1 to 5c can also be achieved by comprising in a disposable absorbent article a resilient insert having the desired shape, e.g. between the backsheet and the absorbent core. The insert can be comprised for example only in the central and rear portions of the absorbent article, where the ridge with the desired profile is to be provided, or can extend along the entire length of the absorbent article, in order to provide its whole shape. The resilient insert can be made of any known suitable material, e.g. absorbent or non absorbent material, and can be produced e.g. by thermoforming to get the desired tridimensional shape, preferably with a constant thickness. The insert can completely provide the tridimensional structure, or can alternatively contribute to create and to maintain said structure in an already shaped absorbent article. The insert can also comprise the cut or slit.

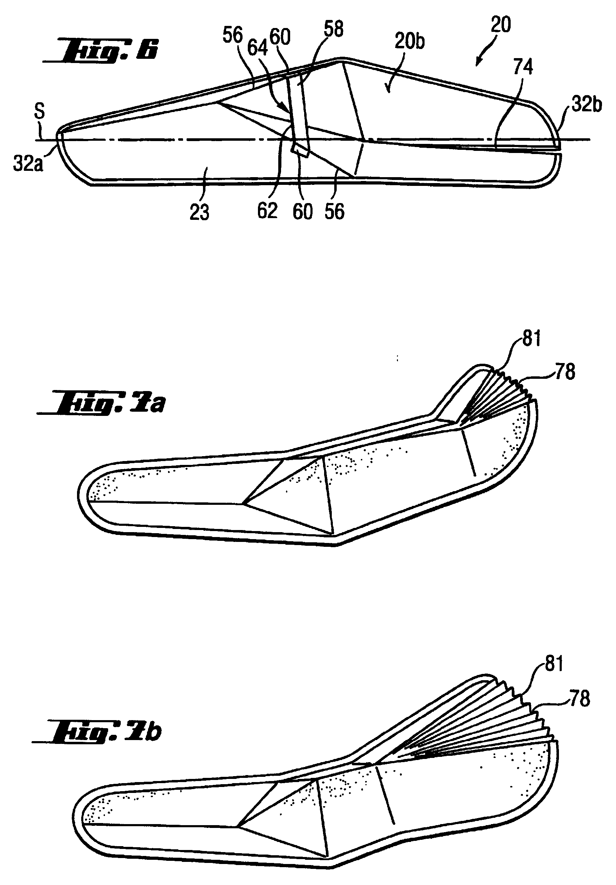

The sanitary napkin 20 according to the present invention and further having the preferred embodiment illustrated in FIGS. 1 to 5c and described hereinbefore is preferably intended to be applied by the user directly to the body, and preferably also comprises means 58 for holding and applying it located on the garment facing surface 20b and being oriented transversely, as those described in European patent application EP 97110734.7. As illustrated in FIG. 6, a perspective view of the sanitary napkin 20 of FIG. 1 is shown, as seen from the side that lies remote from the wearer in use, i.e., with the garment facing surface 20b towards the viewer, and further comprising the means 58. The means 58 for holding and applying the sanitary napkin 20 are also referred to hereinbelow as a handling aid.

Of course the means 58 for holding and applying the sanitary napkin 20 of the present invention are also intended for use by a person taking care of a user, e.g. a nurse, who handles the sanitary napkin 20 and applies it to the user's body.

In the preferred embodiment of FIG. 6 the means 58 for holding and applying the sanitary napkin 20 comprises an elongated strip of elastic film material 58 oriented perpendicularly to the longitudinal symmetry plane S and located on the garment facing surface 20b of the sanitary napkin 20, in correspondence of the central portion 42 of the absorbent core 24, at a position approximately longitudinally intermediate between the front end edge 32a and the rear end edge 32b of the sanitary napkin 20. The strip 58 is affixed to the backsheet 23 at its two spaced apart ends 60 disposed on opposite sides of the symmetry plane S, with an intermediate portion 62 being not joined to said garment facing surface 20b and defining a space 64, intended for the insertion of at least one user's finger for holding and applying the sanitary napkin 20. In the embodiment illustrated in FIG. 6, where the sanitary napkin 20 has the preferred tridimensional shape before use, the space 64 is actually comprised between the intermediate portion 62 of the strip 58 and the garment facing surface 20b of the central portion of the sanitary napkin, which is concave on its garment facing surface 20b, since it corresponds to the ridge 50 on the body facing surface 20a. Typically the spaced apart ends 60 of the strip 58 are fixed with known means, e.g., with an adhesive, or by thermobonding, to the garment facing surface 20b of the backsheet 23 at intermediate locations between each bend line corresponding to the embossments 56, and the respective longitudinal edge 31.

The user can put the sanitary napkin 20 on the palm of her hand with the garment facing surface 20b contacting the hand and with the front end edge 32a facing towards the wrist, at the same time inserting typically one of her fingers, e.g. the middle finger, in the space 64 between the intermediate portion 62 of the strip 58 and the backsheet 23. The user can therefore hold the sanitary napkin 20 in her open hand without exerting any force, also owing to the elasticity of the preferred material that constitutes the strip 58, with substantially the front portion of the sanitary napkin 20 lying on her palm. Application to the body can then be easily performed by the user with a single movement of her open hand, which is simple and self-explanatory as putting the empty hand on the body.

Moreover, the movements of the hand and of the fingers allow the user to completely control the manipulation of the sanitary napkin 20 during its application to the body, making use of the tactile sensitivity of the fingers to find the right position for the sanitary napkin 20. Particularly, in the preferred embodiment of the present invention, the finger inserted in the space 64 is substantially aligned with the ridge 50 on the body facing surface 20a of the sanitary napkin 20, and therefore can provide guidance to control the proper placement of the napkin 20 on the body anatomy, i.e. with the ridge 50 suitably registered with the longitudinal non-linear groove of the female anatomy extending from the labia majora to the gluteal groove. The forward portion of the ridge can be e.g. easily identified by the user with her finger inserted in the space 64, and used as a reference to direct the sanitary napkin into an optimal position on the body. The handling aid constituted by the strip 58 also allows an easy removal of the hand once the sanitary napkin 20 is in place, without disturbing or modifying the position of the napkin 20.

Since in the preferred embodiment of the present invention described so far the tridimensional sanitary napkin 20 does not comprise a panty fastening system, the handling aid of the present invention illustrated in FIG. 6 preferably also allows an easy removal and, possibly, a subsequent reapplication of the sanitary napkin 20 from the body in order to use the toilet, or to make a check of the product, or in any case in order to finally dispose of the product. The user can in fact easily grab the sanitary napkin 20 while it is being worn by positioning her hand substantially in the same way as for the application, with one of her fingers inserted in the space 64 between the not joined portion 62 of the strip 58 and the backsheet 23. The sanitary napkin 20 can therefore be taken off the body and securely held by the user; the handling aid may also be used to temporarily store the sanitary napkin, e.g. while using the toilet, on the user's hand, with no need for actually holding it with the fingers, or for exerting any force on it.

The handling aid constituted by the strip 58 allows in any event the user to handle/manipulate the sanitary napkin 20 by contacting its garment facing surface 20b only, therefore protecting her hand from the possibly dirty body facing surface 20a.

In the preferred absorbent articles having a tridimensional shape before the use, such as the sanitary napkin 20 in the preferred embodiment described hereinbefore, the handling aid preferably also contributes to keep the tridimensional shape of the article during the use, e.g. in case of body movements that can disturb the proper fit of the product, or when in general there is a risk of collapse of the body fitting tridimensional shape. Otherwise the handling aid, e.g. constituted by the strip 58 illustrated in FIG. 6, stays aligned or folded or loose on the garment facing surface 20b of the product and does not disturb the product performance.

In alternative embodiments of the present invention the handling aid can be constituted by more than one strip of material, or by one or more strings, while the material can be also non elastic. The handling aid can be also constituted by a strip arranged as a loop and applied to the garment facing surface 20b of the article, or by a series of loops, intended to allow the insertion of at least one user's finger.

The handling aid can also be activated by the user, e.g. by being applied to the garment facing surface of the absorbent article just before use; alternatively, a handling aid e.g. constituted by a strip 58 can be detached e.g. at one of its ends from the garment facing surface of the absorbent article and then repositioned at a different place, in order to e.g. partially control or adapt a tridimensional shape already provided in the absorbent article, or to modify the space 64 available for the insertion of at least one user's finger. A handling aid preferably constituted by a strip 58 could therefore be resealably attached to the garment facing surface 20b of the absorbent article, at either one or both ends 60, e.g. by means of a resealable adhesive, or of a mechanical fastener of the hook and loop type, such as that marketed under the tradename VELCRO. A handling aid in form of a loop could be modified by the user in order to change the diameter of the loop, and hence the space available for the insertion of the finger(s).

In a further alternative embodiment of the present invention the disposable absorbent article can comprise a release cover releasably attached to the garment facing surface of the absorbent article, wherein the handling aid is located on said release cover. In use, after application of the absorbent article to the body by means of the handling aid, the release cover can be detached from the garment facing surface of the article, leaving the adhesive exposed, that can thus serve as a panty fastening adhesive as it is already known in the art. Successive removal of the absorbent article would be performed e.g. with the known method, using the panty, with the now attached absorbent article, as an handling aid.

The handling aid does not necessarily extend across the entire width of the absorbent article, in order to define a suitable space for the insertion of at least one users finger, which is capable of achieving a sufficiently firm fit with said at least one finger.

As illustrated in the preferred embodiment of FIG. 6, the handling aid does not extend in longitudinal direction over a major portion of the length of the disposable absorbent article; preferably, it extends over less than 10% of said length, being more preferably a narrow strip with a width, extending in said longitudinal direction, of about 1 cm.

The absorbent article of the present invention can be also provided with a panty fastening means, which provides means to attach the article to the undergarment after it has been applied to the body. This would subsequently allow removal of the article from the body in a rather traditional way, i.e. by means of the panty to which the article is adhered. Panty fastening means could be located on a limited portion of the garment facing surface of the absorbent article, in order to avoid the risk of sticking to the user's hand during handling and application of the absorbent article, or, alternatively, it could be activated by the user after the absorbent article has been actually applied to the body, e.g. by removing a release paper. In any case the panty fastening means may comprise a mechanical fastener such as hook and loop fasteners such as marketed under the tradename VELCRO, snaps or holders, which would have the advantage that they do not stick to the user's hand. Alternatively, the absorbent article could be fastened to the undergarment by means of panty fastening adhesive on the backsheet 23. The panty fastening adhesive would provide a means for securing the absorbent article to the panty and preferably a means for securing the absorbent article when soiled to the fold and wrap package for convenient disposal. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive can be typically applied to the backsheet by slot coating or spraying in various distribution patterns, such as e.g. continuous or discontinuous strips, intermittent dots, random patterns spirals.

The panty fastening adhesive should be typically covered with a removable release paper or film in order to prevent the adhesive from drying out or adhering to another surface other than the panty. Any commercially available release paper or film may be used. Suitable examples include BL 30MG-A SILOX EI/O and BL 30 MG-A SILOX 4 P/O available from Akrosil Corporation.

In a further alternative embodiment of the present invention the tridimensional disposable absorbent article can comprise a body adhesive on its body facing surface in order to be adhered directly to the wearer's body, preferably with no need of a panty fastening adhesive.

The tridimensional absorbent articles of the present invention, particularly the sanitary napkin 20, have a length that preferably ranges among the typical values commonly used for different sizes of said sanitary articles intended for substantially external disposition adjacent to the body of the wearer. Particularly, the central and rear portions 42 and 44 of the absorbent core 24 do not have preferably a length which is smaller than the total maximum length of the labia majora of an average user.

The tridimensional absorbent articles of the present invention may further comprise an odour-control material for controlling unpleasant odours associated with absorbed body fluids.

Any known odour-control agent or any combination thereof that can be suitably included in a disposable absorbent article, including other materials such as binders and/or substrates, can be comprised in the absorbent article of the present invention as the odour-control material.

The odour-control material can be incorporated into the absorbent article by methods known in the art, for example layered on or into the absorbent core or mixed within the absorbent core.

In an alternate embodiment of the present invention, the absorbent article may have two flaps (not shown), each of which is adjacent to and extends laterally from the respective side edge of the absorbent core. The flaps are configured to drape over the edges of the wearer's panties in the crotch region so that the flaps are disposed between the edges of the wearers panties and the wearer's thighs. The flaps help serve to prevent soiling of the wearers body and panties by menstrual fluid, preferably by forming a double wall barrier along the edges of the panty. The flaps may be also provided with attachment means on their garment facing surface so that the flaps can be folded back under the panty and attached to the garment facing side of the panty.

The flaps may be constructed of various materials including materials used for the topsheet 22, backsheet 23, combinations thereof, and may be a laminate having tissue in the centre. Further, the flaps may be a separate element attached to the main body of the tridimensional absorbent article or can comprise extensions of the topsheet 22 and/or backsheet 23. It is recommended, however, that the flaps have a liquid impervious backsheet to prevent body fluids which reach the flaps from soiling the edges of the wearer's panties.

Preferred flaps that are suitable or adaptable to the tridimensional absorbent article of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, but preferably over the above mentioned flaps, the tridimensional absorbent article may comprise components that naturally wrap the sides of a wearers panties. Sanitary napkins having components that naturally wrap the sides of a wearer's panties suitable for use with the tridimensional absorbent article of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

Although the disposable absorbent article of the present invention has been described with reference to a sanitary napkin, it can be used beneficially in the context of other disposable absorbent articles such as panty liners and incontinence articles. The disposable absorbent article may thus also have all those features and parts which are typical for products in the context of their intended use.

## Claims

1. A disposable absorbent article adapted to form a three dimensional shape before use, said disposable absorbent article comprising a liquid pervious topsheet, a liquid impervious backsheet, and an absorbent core intermediate said topsheet and said backsheet,
said absorbent core having a central acquisition region and a peripheral region external to said central acquisition region,
said absorbent core comprising a fluid storage layer,
said disposable absorbent article being capable of acquiring body fluids through said topsheet in said central acquisition region of said absorbent core,
said disposable absorbent article characterized in that
said absorbent core comprises in said peripheral region a material distinct from said fluid storage layer, said material being a high void volume fluid handling material.

2. A disposable absorbent article according to claim 1, characterized in that said absorbent core comprises a fluid storage layer in fluid communication with said high void volume fluid handling material, wherein said high void volume fluid handling material is comprised between said topsheet and said storage layer, said high void volume fluid handling structure having a thickness such as to separate said topsheet from said fluid storage layer.

3. A disposable absorbent article according to any preceding claim, characterized in that said absorbent core has two longitudinal side edges and a side region along each of said longitudinal side edges, said side region comprised in said peripheral region, wherein said high volume fluid handling material is comprised in each of said side regions.

4. A disposable absorbent article according to any preceding claim, characterized in that said fluid handling material has a bulkiness of at least 15 cm³/g, preferably of from 20 cm³/g to 100 cm³/g, more preferably of from 30 cm³/g to 50 cm³/g, as measured under a pressure of 2 kPa.

5. A disposable absorbent article according to any preceding claim, characterized in that said high void volume fluid handling material has a thickness of from 1 mm to 5 mm, preferably of from 1.5 mm to 2.5 mm.

6. A disposable absorbent article according to any of claims 2 to 5, characterized in that said core comprises a fluid acquisition layer which is at least partially coextensive with said fluid storage layer and with said high void volume fluid handling material, being intermediate said topsheet and said fluid storage layer, and in fluid communication therewith, in said central region, and intermediate said high void volume fluid handling material and said fluid storage layer, and in fluid communication therewith, in at least part of said peripheral region.

7. A disposable absorbent article according to claim 6, characterized in that said core further comprises a fluid distribution layer extending at least in said central region, said fluid distribution layer being intermediate said fluid acquisition layer and said storage layer and in fluid communication therewith, said fluid distribution layer having a density which is higher than the density of said fluid handling material.

8. A disposable absorbent article according to any preceding claim, characterized in that said high void volume fluid handling structure is adapted to release to the fluid storage layer substantially all the fluid which it receives.

9. A disposable absorbent article according to any preceding claim, characterized in that said article has a tridimensional shape prior to use and is further provided with a structural tridimensionality, wherein said article cannot be completely flattened onto a flat surface while keeping its integrity.
